# EUROPEAN PATENT APPLICATION

(11) **EP 3 991 799 A1**
(43) Date of publication of application: **04.05.2022**
(21) Application number: 20831884.0
(22) Date of filing: 12.06.2020
(51) Int. Cl.: A61Q 19/00, C08K 5/05, C08K 5/06, C08L 33/02, C08L 67/00, C08L 91/00, A61K 8/06, A61K 8/34, A61K 8/60, A61K 8/73, A61K 8/81, A61K 8/86, A61K 8/92

(54) **VISCOUS COMPOSITION**

(30) Priority: 26.06.2019 JP 2019118781
(71) Applicant: Sumitomo Seika Chemicals Co., Ltd., Kako-gun, Hyogo 675-0145 (JP)
(72) Inventor: YAMAGUCHI, Hirofumi, Himeji-shi, Hyogo 672-8076 (JP)
(74) Representative: Vossius & Partner Patentanwälte Rechtsanwälte mbB
(86) International application number: PCT/JP2020/023218
(87) International publication number: WO 2020/262043

(57) **Abstract**

Provided is a viscous emulsion composition that contains a relatively high concentration of a carboxyl-group-containing water-soluble copolymer but is not so viscous as to lose handleability.

## Description

### Technical Field

The present disclosure relates to a viscous composition etc. More specifically, the present disclosure relates to a viscous composition that contains a carboxyl-group-containing water-soluble copolymer. The entire contents of the documents disclosed in the present specification are incorporated herein by reference.

### Background Art

Water-soluble copolymers containing carboxyl groups are used in various fields as a thickening agent for cosmetics etc., a moisturizer for poultices etc., a suspension stabilizer for emulsions, suspensions, etc., and a gelling base for batteries etc.

### Citation List

### Patent Literature

PTL 1: WO 2014/021434
PTL 2: JP2013-63959A

### Summary of Invention

### Technical Problem

Carboxyl-group-containing water-soluble copolymers have a thickening effect. By dissolving or dispersing a carboxyl-group-containing water-soluble copolymer in, for example, water, a viscous composition can be obtained. The thickening effect drastically increases with an increase in the amount of the copolymer; thus, water that contains a large amount of the copolymer has an overly high viscosity or is in the form of a nearly solid, and has significantly reduced handleability. Therefore, a viscous composition that contains 1 to 1.5 mass%, or at most about 2 mass%, of a carboxyl-group-containing water-soluble copolymer is typically prepared for use in the production of subsequent products (e.g., cosmetics).

On the other hand, a higher concentration of an aqueous dispersion composition obtained by dissolving or dispersing the copolymer in water increases efficiency more in the production of products such as cosmetics. Therefore, it would be advantageous if it is possible to prepare a viscous composition that comprises the copolymer in a high concentration, but has intact handleability.

### Solution to Problem

The present inventor found the possibility that a combined use of a specific carboxyl-group-containing water-soluble copolymer and a specific emulsifier enables the production of a viscous emulsion composition that contains a relatively high concentration of the copolymer but is not so viscous as to lose handleability. Based on this finding, the inventor made further research.

The present disclosure encompasses, for example, the subject matter described in the following items.

### Item 1.

A viscous emulsion composition comprising:
(A) at least one copolymer selected from the group consisting of the following (A-1), (A-2), and (A-3), or a salt thereof:
   (A-1) a copolymer obtained by polymerizing the following (i) and (ii),
   (A-2) a copolymer obtained by polymerizing the following (i) and (iii), and
   (A-3) a copolymer obtained by polymerizing the following (i), (ii), and (iii):
      (i) 100 parts by mass of a (meth)acrylic acid,
      (ii) 0.001 to 1 part by mass of a compound with two or more ethylenically unsaturated groups, and
      (iii) 0.5 to 5 parts by mass of a (meth)acrylic acid alkyl ester having an alkyl group with 10 to 30 carbon atoms;
(B) at least one emulsifier selected from the group consisting of the following (B-1), (B-2), and (B-3):
   (B-1) an alcohol having a structure in which at least one hydrogen atom of a linear alkane with 18 to 24 carbon atoms is substituted with a hydroxymethyl group or a hydroxyethyl group,
   (B-2) an ether of the alcohol (B-1) with a monosaccharide or a polysaccharide of 2 to 6 monosaccharides, and
   (B-3) a polyethylene glycol (mono- or di-) ester of a polyhydroxy fatty acid;
(C) water; and
(D) an oil component,
   wherein
(A) is present in an amount of 10 to 35 mass%, and the mass ratio ((D)/(B)) of (D) to (B) is 1 to 8.

### Item 2.

The composition according to Item 1, wherein the mass ratio ((D)/(B)) of (D) to (B) is 2 to 5.

### Item 3.

The composition according to Item 1 or 2, comprising (D) in an amount of 20 to 50 mass%.

### Item 4.

The composition according to any one of Items 1 to 3, wherein the composition has a viscosity at 25°C of 1000 to 100000 mPa·s.

### Item 5.

The composition according to any one of Items 1 to 4, wherein the composition satisfies at least one of the following requirements (α) to (γ):
(α) (B-1) is octyldodecanol,
(β) the saccharide in (B-2) is xylose, and
(γ) (B-3) is a polyethylene glycol mono- or diester of polyhydroxystearic acid.

### Item 6.

The composition according to any one of Items 1 to 4, wherein
(B-1) is octyldodecanol,
(B-2) is octyldodecyl xyloside, and
(B-3) is a polyethylene glycol diester of polyhydroxystearic acid.

### Item 7.

The composition according to any one of Items 1 to 6, wherein (A) is selected from the group consisting of the copolymer (A-1) or a salt thereof, and the copolymer (A-3).

### Item 8.

The composition according to any one of Items 1 to 7, wherein (B) comprises (B-1), (B-2), and (B-3).

### Item 9.

The composition according to any one of Items 1 to 7, wherein (B) comprises 50 to 60 mass% of (B-1), 10 to 30 mass% of (B-2), and 10 to 30 mass% of (B-3).

### Item 10.

A viscous emulsion composition comprising:
(A) at least one copolymer selected from the group consisting of the following (A-1) and (A-3), or a salt thereof:
   (A-1) a copolymer obtained by polymerizing the following (i) and (ii), and
   (A-3) a copolymer obtained by polymerizing the following (i), (ii), and (iii):
      (i) 100 parts by mass of a (meth)acrylic acid,
      (ii) 0.001 to 1 part by mass of a compound with two or more ethylenically unsaturated groups, and
      (iii) 0.5 to 5 parts by mass of a (meth)acrylic acid alkyl ester having an alkyl group with 10 to 30 carbon atoms;
(B) an emulsifier comprising 50 to 60 mass% of the following (B-1), 10 to 30 mass% of the following (B-2), and 10 to 30 mass% of the following (B-3):
   (B-1): octyldodecanol,
   (B-2): octyldodecyl xyloside, and
   (B-3): a polyethylene glycol diester of polyhydroxystearic acid;
(C) water; and
(D) an oil component,
   wherein
   (A) is present in an amount of 10 to 35 mass%, while (D) is present in an amount of 20 to 50 mass%, and the mass ratio ((D)/(B)) of (D) to (B) is 1 to 8.

### Advantageous Effects of Invention

Provided is a viscous emulsion composition that contains a relatively high concentration of a carboxyl-group-containing water-soluble copolymer but is not so highly viscous as to lose handleability.

### Description of Embodiments

Embodiments encompassed by the present disclosure are described in more detail below. The present disclosure preferably encompasses, for example, a viscous composition (in particular, a viscous composition that comprises a carboxyl-group-containing water-soluble copolymer, and that can achieve maintained handleability), and a method for preparing the viscous composition. However, the present disclosure is not limited to these and encompasses everything stated in this specification and recognizable to those skilled in the art.

The viscous emulsion composition encompassed by the present disclosure comprises (A) a specific copolymer or a salt thereof, (B) a specific emulsifier, (C) water, and (D) an oil component. This viscous emulsion composition may be referred to below as "the viscous emulsion composition according to the present disclosure."

The specific copolymer or a salt thereof (A) is at least one copolymer selected from the group consisting of the following (A-1), (A-2), and (A-3), or a salt thereof.

(A-1) a copolymer obtained by polymerizing the following (i) and (ii),
(A-2) a copolymer obtained by polymerizing the following (i) and (iii), and
(A-3) a copolymer obtained by polymerizing the following (i), (ii), and (iii):
   (i) 100 parts by mass of a (meth)acrylic acid,
   (ii) 0.001 to 1 part by mass of a compound with two or more ethylenically unsaturated groups, and
   (iii) 0.5 to 5 parts by mass of a (meth)acrylic acid alkyl ester having an alkyl group with 10 to 30 carbon atoms.

The copolymers (A-1), (A-2), and (A-3) above may be referred to as "the copolymer (A-1)," "the copolymer (A-2)," and "the copolymer (A-3)," respectively. Further, the copolymers (A) (i.e., the copolymers (A-1) to (A-3) collectively) may be referred to as "the copolymers of the present disclosure." The copolymers of the present disclosure are more preferably (A-1) or (A-3) .

In this specification, the term "(meth)acrylic" means acrylic and/or methacrylic. For the (meth)acrylic acid, either acrylic acid or methacrylic acid may be used alone or used in combination.

The (meth)acrylic acid alkyl ester having an alkyl group with 10 to 30 (10, 11, 12, 13, 14, 15, 16, 17, 18, 19, 20, 21, 22, 23, 24, 25, 26, 27, 28, 29, or 30) carbon atoms refers to an ester of (meth)acrylic acid and a higher alcohol having an alkyl group with 10 to 30 carbon atoms. The carbon number of this alkyl group is more preferably, for example, 12 to 30, 14 to 28, 16 to 26, or 18 to 24. Examples of the (meth)acrylic acid alkyl ester include, but are not particularly limited to, esters of (meth)acrylic acid with stearyl alcohol, esters of (meth)acrylic acid with eicosanol, esters of (meth)acrylic acid with behenyl alcohol, and esters of (meth)acrylic acid with tetracosanol. Among these (meth)acrylic acid alkyl esters, stearyl methacrylate, eicosanyl methacrylate, behenyl methacrylate, and tetracosanyl methacrylate are preferred. These (meth)acrylic acid alkyl esters may be used alone, or in a combination of two or more. These (meth)acrylic acid alkyl esters for use may be commercial products, such as BLEMMER VMA-70 and BLEMMER SMA, product names, produced by NOF Corporation.

The amount of the (meth)acrylic acid alkyl ester for use is 0.5 to 10 parts by mass, preferably 0.5 to 5 parts by mass or 1 to 5 parts by mass, and more preferably 1 to 3 parts by mass, per 100 parts by mass of the (meth)acrylic acid.

Examples of the compound with two or more ethylenically unsaturated groups include, but are not particularly limited to, a compound in which a polyol, such as ethylene glycol, propylene glycol, polyoxyethylene glycol, polyoxypropylene glycol, glycerol, polyglycerol, trimethylolpropane, pentaerythritol, saccharose, or sorbitol, is substituted with two or more acrylic acid esters; a compound in which a polyol mentioned above is substituted with two or more allyl ethers; and diallyl phthalate, triallyl phosphate, allyl methacrylate, tetraallyloxyethane, triallyl cyanurate, divinyl adipate, vinyl crotonate, 1,5-hexadiene, divinyl benzene, and polyallylsaccharose. Among these, the compound with two or more ethylenically unsaturated groups is preferably sucrose allyl ether, pentaerythritol allyl ether (more preferably pentaerythritol triallyl ether or pentaerythritol tetraallyl ether), tetraallyloxyethane, triallyl phosphate, or polyallylsaccharose, and more preferably sucrose allyl ether or pentaerythritol allyl ether. These compounds with two or more ethylenically unsaturated groups may be used alone, or in a combination of two or more.

The amount of the compound with two or more ethylenically unsaturated groups for use is 0.001 to 1 part by mass per 100 parts by mass of the (meth)acrylic acid. The lower limit of this range may be, for example, 0.005, 0.01, 0.015, 0.02, 0.025, 0.03, 0.035, or 0.04. The upper limit of this range may be, for example, 0.95, 0.9, 0.85, 0.8, 0.75, 0.7, 0.65, or 0.6. For example, the amount for use is preferably 0.01 to 0.8 parts by mass, and more preferably 0.02 to 0.7 parts by mass, per 100 parts by mass of the (meth)acrylic acid.

In the copolymer (A-1), in particular, the amount of the compound with two or more ethylenically unsaturated groups for use is preferably 0.1 to 1 part by mass per 100 parts by mass of the (meth)acrylic acid. The upper or lower limit of the mass ratio range may be 0.2, 0.3, 0.4, 0.5, 0.6, 0.7, 0.8, or 0.9. For example, the mass ratio range is more preferably 0.2 to 0.8 or 0.3 to 0.7.

In the copolymer (A-3), in particular, the amount of the compound with two or more ethylenically unsaturated groups for use is preferably 0.01 to 0.1 parts by mass per 100 mass parts of the (meth)acrylic acid. The upper or lower limit of the mass ratio range may be 0.02, 0.03, 0.04, 0.05, 0.06, 0.07, 0.08, or 0.09. For example, the mass ratio range is more preferably 0.02 to 0.08 or 0.03 to 0.07.

In the production of the copolymers of the present disclosure, the method of polymerizing (i) and (ii), or (i) and (iii), or (i), (ii), and (iii), is not particularly limited. Examples include a method of polymerizing these in a polymerization solvent in the presence of a radical polymerization initiator.

Examples of the radical polymerization initiator include, but are not particularly limited to, α,α'-azobisisobutyronitrile, 2,2'-azobis-2,4-dimethylvaleronitrile, 2,2'-azobis methyl isobutyrate, 2,2'-azobis(2-methylpropionamidine)dihydrochloride, benzoyl peroxide, lauroyl peroxide, cumene hydroperoxide, and tertiary butyl hydroperoxide. These radical polymerization initiators may be used alone, or in a combination of two or more.

The amount of the radical polymerization initiator for use is not particularly limited and is preferably, for example, 0.01 to 0.45 parts by mass, and more preferably 0.01 to 0.35 parts by mass, per 100 parts by mass of the (meth)acrylic acid.

The description for the copolymer (A-1) and the description for the copolymer (A-3) are in common except for the presence of (iii) above during polymerization, unless otherwise noted. Further, the description for the copolymer (A-2) and the description for the copolymer (A-3) are in common except for the presence of (ii) above during polymerization, unless otherwise noted. The description for the copolymer (A-3) in which (iii) above is not used corresponds to the description for the copolymer (A-1), while the description for the copolymer (A-3) in which (ii) above is not used corresponds to the description for the copolymer (A-2).

All of the copolymers (A-1) to (A-3) can be preferably prepared, for example, by precipitation polymerization or reversed-phase suspension polymerization. Further, reversed-phase suspension polymerization may be performed in a polymerization solvent that contains a nonionic surfactant with one or more polyoxyethylene chains.

Preferable examples of the nonionic surfactant with one or more polyoxyethylene chains include polyhydric alcohol fatty acid ester-ethylene oxide adducts, block copolymers of hydroxy fatty acids and ethylene oxide, and polyoxyethylene castor oil.

Preferable examples of the polyhydric alcohol fatty acid ester-ethylene oxide adducts include ester compounds of polyoxyethylene hydrogenated castor oil and polyhydric alcohol fatty acids. The polyhydric alcohol fatty acid here is preferably a saturated or unsaturated polyhydric (in particular, divalent) alcohol fatty acid with 14 to 24 (14, 15, 16, 17, 18, 19, 20, 21, 22, 23, or 24) carbon atoms. More specifically, for example, isopalmitic acid, isostearic acid, and isooleic acid are preferred. The average number of moles of ethylene oxide added to constitute polyoxyethylene in ester compounds of polyoxyethylene hydrogenated castor oil and polyhydric alcohol fatty acids is not particularly limited, and may be, for example, about 20 to 100 or about 30 to 70. Particularly preferable examples of the ester compounds of polyoxyethylene hydrogenated castor oil and polyhydric alcohol fatty acids include polyoxyethylene hydrogenated castor oil isostearates.

In polyoxyethylene castor oil, the number of moles of ethylene oxide added is preferably about 2 to 10, and more preferably about 2 to 5.

The block copolymer of a hydroxy fatty acid and ethylene oxide can be rephrased as a copolymer of a polyhydroxy fatty acid and polyoxyethylene. The fatty acid of polyhydroxy fatty acid is preferably a fatty acid with 14 to 22 carbon atoms. Preferable examples include myristic acid, palmitic acid, and stearic acid. The hydroxy fatty acid is preferably, for example, hydroxymyristic acid, hydroxypalmitic acid, or hydroxystearic acid, and particularly preferably hydroxystearic acid. The hydroxystearic acid is particularly preferably 12-hydroxystearic acid. The polyhydroxy fatty acid is particularly preferably a polyhydroxystearic acid. The block copolymer of a hydroxy fatty acid and ethylene oxide is particularly preferably a block copolymer of 12-hydroxystearic acid and ethylene oxide.

The nonionic surfactant with one or more polyoxyethylene chains can be used alone, or in a combination of two or more.

The amount of the nonionic surfactant with one or more polyoxyethylene chains for use is preferably about 0.5 to 10 parts by mass per 100 parts by mass of the (meth)acrylic acid (i). The lower limit of the range based on parts by mass may be, for example, about 0.75, 1.0, 1.25, or 1.5; and the upper limit of the range based on parts by mass may be, for example, about 9.5, 9, 8.5, 8, 7.5, 7, 6.5, 6, or 5.5. For example, the amount is more preferably about 1 to 7.5 parts by mass.

The polymerization solvent is not particularly limited, and is preferably a solvent that dissolves (meth)acrylic acid, the (meth)acrylic acid alkyl ester, and the compound with two or more ethylenically unsaturated groups, and that does not dissolve the resulting alkyl-modified carboxyl-group-containing water-soluble copolymer. Specific examples of such polymerization solvents include normal pentane, normal hexane, normal heptane, normal octane, isooctane, cyclopentane, methylcyclopentane, cyclohexane, methylcyclohexane, benzene, toluene, xylene, chlorobenzene, ethylene dichloride, ethyl acetate, isopropyl acetate, ethyl methyl ketone, and isobutyl methyl ketone. Among these polymerization solvents, ethylene dichloride, normal hexane, normal heptane, and ethyl acetate are preferred from the standpoint of stable quality and easy availability. These polymerization solvents may be used alone, or in a combination of two or more.

The amount of the polymerization solvent for use is not particularly limited, and is preferably, for example, 200 to 10,000 parts by mass, and more preferably 300 to 2,000 parts by mass, per 100 parts by mass of the (meth)acrylic acid. The use of the polymerization solvent within this range suitably suppresses aggregation of the alkyl-modified carboxyl-group-containing water-soluble copolymer even when the polymerization reaction proceeds, allowing stirring to be performed uniformly, and the polymerization reaction to proceed more efficiently.

The atmosphere during the polymerization reaction is not particularly limited as long as the polymerization reaction can proceed. Examples include an inert gas atmosphere, such as nitrogen gas or argon gas.

The reaction temperature during the polymerization reaction is not particularly limited as long as the polymerization reaction can proceed, and is preferably, for example, 50 to 90°C, and more preferably 55 to 75°C. When the polymerization reaction is performed within this reaction temperature range, an increase in the reaction solution viscosity can be suitably suppressed, and the reaction can be more easily controlled; additionally, the bulk density of the resulting alkyl-modified carboxyl-group-containing water-soluble copolymer can be suitably controlled.

The reaction time for the polymerization reaction cannot be generalized because it varies depending on the reaction temperature. The reaction time is typically 2 to 10 hours.

After completion of the reaction, for example, the reaction solution is heated to 80 to 130°C, and the polymerization solvent is removed, whereby a white fine powder of an alkyl-modified carboxyl-group-containing water-soluble copolymer can be isolated.

In the viscous emulsion composition according to the present disclosure, the copolymers of the present disclosure may be used alone, or in a combination of two or more.

The viscous emulsion composition according to the present disclosure comprises the copolymers of the present disclosure in an amount of 10 to 35 mass%. The upper or lower limit of this range may be, for example, 11, 12, 13, 14, 15, 16, 17, 18, 19, 20, 21, 22, 23, 24, 25, 26, 27, 28, 29, 30, 31, 32, 33, or 34 mass%. For example, the range may be 15 to 35 mass% or 20 to 33 mass%.

As described above, the emulsifier (B) contained in the viscous emulsion composition according to the present disclosure is at least one member selected from the group consisting of the following (B-1), (B-2), and (B-3). The emulsifier (B) may be referred to as "the emulsifier of the present disclosure."

(B-1) an alcohol having a structure in which at least one hydrogen atom of a linear alkane with 18 to 24 (18, 19, 20, 21, 22, 23, or 24) carbon atoms is substituted with a hydroxymethyl group or a hydroxyethyl group,
(B-2) an ether of the alcohol (B-1) with a monosaccharide or a polysaccharide of 2 to 6 monosaccharides, and
(B-3) a polyethylene glycol (mono- or di-) ester of a polyhydroxy fatty acid.

In (B-1), the at least one hydrogen atom substituted with a hydroxymethyl group or hydroxyethyl group may be a hydrogen atom that is bound to any carbon atom of the linear alkane with 18 to 24 carbon atoms. That is, the at least one hydrogen atom may be a hydrogen atom in the -CH₃ group or the - CH₂- group of the alkane. The at least one hydrogen atom may also be a hydrogen atom that is bound to a carbon atom present at one end of the alkane (i.e., the carbon atom in -CH₃ group) or a hydrogen atom that is bound to the 2^{nd}, 3^{rd}, 4^{th}, 5^{th}, 6^{th}, 7^{th}, 8^{th}, 9^{th}, 10^{th}, 11^{th}, 12^{th}, 13^{th}, 14^{th}, 15^{th}, 16^{th}, 17^{th}, 18^{th}, 19^{th}, 20^{th}, 21^{st}, 22^{nd}, 23^{rd}, or 24^{th} carbon atom from one end of the alkane. The number of the substituted hydrogen atoms is preferably, for example, 1, 2, 3, or 4, and particularly preferably 1.

In particular, the alcohol (B-1) is preferably octyldodecanol (in particular, 2-octyldodecanol), and more preferably 2-n-octyl-1-dodecanol.

The monosaccharide constituting the ether (B-2) is preferably a pentose or a hexose, and more preferably a pentose. Further, the monosaccharide is preferably an aldose or a ketose, and more preferably an aldose. More specifically, the monosaccharide is particularly preferably xylose.

The alcohol (B-1) constituting the ether (B-2) and the alcohol (B-1) contained in the viscous emulsion composition according to the present disclosure may be the same or different.

The ether (B-2) is more specifically a compound in which a monosaccharide or a polysaccharide of 2 to 6 (2, 3, 4, 5, or 6) monosaccharides and the alcohol (B-1) are linked by a glycosidic linkage, and is represented by the following formula (I) :

R-O-(X)p (I),

wherein X is identical or different, and each represents a saccharide residue, p is 1 to 6, and R-O- represents a group having a structure in which the hydrogen atom is removed from the OH group of the alcohol (B-1). The oligomeric structure (X)p may represent any isomer of either a constitutional isomer or a stereoisomer, or may otherwise represent a mixture of these isomers.

In formula (I), p represents the average polymerization degree of saccharide and is a number of 1 to 6, preferably 1 to 2.5, and more preferably 1 to 2.0.

In particular, the ether (B-2) is preferably octyldodecyl xyloside. In the present disclosure, octyldodecyl xyloside refers to a compound represented by formula (I) above, wherein R-O- represents a group in which the hydrogen atom is removed from the OH group of octyldodecanol, X is the same, and each represents xylose, and p (average polymerization degree of xylose) is about 1 to 2.5.

The fatty acid in the polyhydroxy fatty acid that constitutes the ester (B-3) is preferably a saturated or unsaturated fatty acid with 12 to 24 (12, 13, 14, 15, 16, 17, 18, 19, 20, 21, 22, 23, or 24) carbon atoms. The fatty acid is preferably a straight-chain or branched-chain fatty acid, and more preferably a straight-chain fatty acid. More specifically, lauric acid, myristic acid, palmitic acid, stearic acid, oleic acid, linoleic acid, and the like are preferred, and stearic acid is particularly preferred. A hydroxy fatty acid has a structure in which at least one hydrogen atom that is bound to the carbon chain constituting the fatty acid is substituted with a hydroxy group. The hydrogen atom that is substituted may be a hydrogen atom that is bound to any carbon atom other than the carbon atom of the carboxyl group. For example, the hydrogen atom may be a hydrogen atom that is bound to the 2^{nd}, 3^{rd}, 4^{th}, 5^{th}, 6^{th}, 7^{th}, 8^{th}, 9^{th}, 10^{th}, 11^{th}, 12^{th}, 13^{th}, 14^{th}, 15^{th}, 16^{th}, 17^{th}, 18^{th}, 19^{th}, 20^{th}, 21st, 22^{nd}, or 23^{rd} carbon atom in the carbon chain of the fatty acid, counting from the carbon atom of the carboxyl group (which is taken as the first). The number of hydrogen atoms substituted (i.e., the number of the hydroxy groups in the hydroxy fatty acid) may be, for example, 1, 2, 3, or 4, and particularly preferably 1. The polyhydroxy fatty acid preferably contains the same or different hydroxy fatty acids, and more preferably contains the same hydroxy fatty acids. Of these, the hydroxy fatty acid is preferably a hydroxystearic acid, and particularly preferably 12-hydroxystearic acid. The polyhydroxy fatty acid preferably has an average molecular weight of 1000 to 4000.

More specifically, the polyhydroxy fatty acid that constitutes the ester (B-3) is preferably a polyhydroxystearic acid (in particular, poly(12-hydroxystearic acid)). The polyhydroxystearic acid preferably has an average molecular weight of 1000 to 4000.

The polyethylene glycol that constitutes the ester (B-3) preferably has an average molecular weight of about 400 to 6000.

Specifically, the ester (B-3) is preferably a polyethylene glycol mono- or diester of polyhydroxystearic acid, and more preferably a polyethylene glycol diester of polyhydroxystearic acid. These diesters are preferably those with the INCI name "PEG-30 dipolyhydroxystearate." Examples of commercial products of such diesters include Arlacel P-135 (product name) produced by Uniqema.

(B), i.e., the emulsifier of the present disclosure, preferably comprises (B-1), (B-2), and (B-3), and in particular comprises 50 to 60 mass% of (B-1), 10 to 30 mass% of (B-2), and 10 to 30 mass% of (B-3). (B) for use is preferably, for example, EASYNOV (product name) produced by Seppic. EASYNOV is a composition comprising 50 to 60 mass% of 2-octyldodecanol, 15 to 25 mass% of 2-octyldodecyl xyloside, and 15 to 25 mass% of PEG-30 dipolyhydroxystearate.

The viscous emulsion composition according to the present disclosure preferably comprises the emulsifier (B) in an amount of, for example, 7 to 30 mass%. The upper or lower limit of this range may be, for example, 8, 9, 10, 11, 12, 13, 14, 15, 16, 17, 18, 19, 20, 21, 22, 23, 24, 25, 26, 27, 28, or 29 mass%. For example, the range may be 8 to 20 mass% or 9 to 15 mass%.

The viscous emulsion composition according to the present disclosure comprises water (C) and an oil component (D).

Water for use is not particularly limited, and may be, for example, ion-exchanged water or distilled water.

The oil component is also not particularly limited, and various oil components are usable. For example, well-known oil components used in the field of cosmetics are usable. Specific examples of the oil component include olive oil, macadamia ternifolia seed oil, camellia oil, meadowfoam oil, sunflower oil, safflower oil, castor oil, jojoba oil, carnauba wax, candelilla wax, lanolin, rice bran wax, liquid paraffin, isoparaffin, squalane, Vaseline, ceresin, isopropyl myristate, glycerol tri-2-ethylhexanoate, glycerol triisostearate, glycerol triisopalmitate, glycerol tricaprylate, glycerol tricaprate, pentaerythritol tetra 2-ethylhexanoate, trimethylolpropane tri-2-ethylhexanoate, diisostearyl malate, 2-ethylhexyl palmitate, cetyl 2-ethylhexanoate, isostearyl isostearate, dimethyl polysiloxane, methyl phenyl polysiloxane, decamethylcyclopentasiloxane, ceramides, and silicone oils.

Of the oil components, more preferable examples include isopropyl myristate, glyceryl tri(caprylate/caprate), jojoba oil, and squalane.

The viscous emulsion composition according to the present disclosure preferably comprises (D) in an amount of 20 to 50 mass%. The upper or lower limit of this amount range may be, for example, 21, 22, 23, 24, 25, 26, 27, 28, 29, 30, 31, 32, 33, 34, 35, 36, 37, 38, 39, 40, 41, 42, 43, 44, 45, 46, 47, 48, or 49 mass%. For example, the amount range is more preferably 25 to 50 mass% or 30 to 45 mass%.

In the viscous emulsion composition according to the present disclosure, the mass ratio ((D)/(B)) of (D) to (B) is preferably 1 to 8. The upper or lower limit of the mass ratio range may be, for example 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, or 7.9. For example, the mass ratio range is more preferably 2 to 5, 2.5 to 5, 3 to 5, or 3.5 to 4.5.

The viscous emulsion composition according to the present disclosure preferably has a viscosity (measured at 25°C) of 1000 to 100000 mPa·s. The upper or lower limit of the viscosity range may be, for example, 1500, 2000, 2500, 3000, 3500, 4000, 4500, 5000, 5500, 6000, 6500, 7000, 7500, 8000, 8500, 9000, or 9500 mPa·s. For example, the viscosity range is more preferably 2000 to 9500 mPa·s or 3000 to 7500 mPa·s.

The viscosity as used herein refers to a value obtained by measuring the viscosity of an evaluation sample (a viscous emulsion composition) with an adjusted temperature of 25°C (for example, adjusted by immersing in a constant-temperature water tank for 60 minutes or more) using a Brookfield viscometer (model number: DV1MRVTJ0) at 25°C after 1-minute rotation at a rotation speed of 20 revolutions per minute. For rotors used in the measurement, a No. 3 rotor is used for viscosity of less than 2000 mPa·s, a No. 4 rotor is used for viscosity of 2000 mPa·s or more and less than 5000 mPa·s, a No. 5 rotor is used for viscosity of 5000 mPa·s or more and less than 15000 mPa·s, a No. 6 rotor is used for viscosity of 15000 mPa·s or more and less than 40000 mPa·s, and a No. 7 rotor is used for viscosity of 40000 mPa·s or more and less than 180000 mPa·s.

The viscous emulsion composition according to the present disclosure is preferably a W/O emulsion composition.

In this specification, the terms "comprising" and "containing" include consisting essentially of and consisting of. Further, the present invention includes any combination of the constituent requirements described in this specification.

In addition, the various characteristics (properties, structures, functions, etc.) described in each embodiment of the present invention described above may be combined in any way in specifying the subjects included in the present invention. In other words, the present invention includes all the subjects comprising all combinations of the combinable characteristics described in this specification.

### Examples

The present invention is described in more detail below. However, the present invention is not limited to the following Examples.

### Synthesis of (Meth)acrylic Acid Copolymer

### Production Example 1

45 g (0.625 mol) of acrylic acid, 1.35 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate), 0.02 g of pentaerythritol tetraallyl ether, 150 g of normal hexane, and 0.081 g (0.00035 mol) of 2,2'-azobis methyl isobutyrate were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the solvent. Then, the mixture was reacted for 4 hours in a nitrogen atmosphere while the temperature was maintained at 60 to 65°C. After completion of the reaction, the resulting slurry was heated to 90°C to distill off the normal hexane, and further dried under reduced pressure for 8 hours (110°C, 10 mmHg) to thus obtain 43 g of a white fine powder of an alkyl-modified carboxyl-group-containing water-soluble copolymer. This copolymer is sometimes referred to below as "the polymer of Production Example 1."

### Production Example 2

45 g (0.625 mol) of acrylic acid, 0.27 g of pentaerythritol tetraallyl ether, 150 g of n-hexane, and 0.081 g (0.00035 mol) of 2,2'-azobis methyl isobutyrate were placed in a 500-mL four-necked flask equipped with a stirrer, a thermometer, a nitrogen blowing tube, and a cooling tube to prepare a reaction liquid. After the reaction liquid was stirred and mixed uniformly, nitrogen gas was blown into the solution to remove oxygen present in the upper space of the reaction vessel, and in the raw material and the solvent. Thereafter, the reaction liquid was reacted for 4 hours in a nitrogen atmosphere while the temperature was maintained at 60 to 65°C. After completion of the reaction, the resulting slurry was heated to 90°C to distill off the n-hexane, and further dried under reduced pressure for 8 hours (110°C, 10 mmHg) to thus obtain 42 g of a carboxyvinyl polymer. This carboxyvinyl polymer is sometimes referred to below as "the polymer of Production Example 2."

### Production Example 3

40 g of acrylic acid, 0.4 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.19 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 1.6 g of a block copolymer of 12-hydroxystearic acid and polyoxyethylene (Hypermer B246, produced by Croda) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 39 g of a white fine powder of an alkyl-modified carboxyl-group-containing water-soluble copolymer. This copolymer is sometimes referred to below as "the polymer of Production Example 3."

### Production Example 4

40 g of acrylic acid, 0.88 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 0.26 g of polyoxyethylene castor oil (produced by Nikko Chemicals Co., Ltd., product name: CO-3, an adduct in which 3 mol of ethylene oxide is added), and 0.98 g of polyoxyethylene hydrogenated castor oil triisostearate (Nihon Emulsion Co., Ltd., product name: RWIS-350, an adduct in which 50 mol of ethylene oxide is added) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 38 g of a white fine powder of an alkyl-modified carboxyl-group-containing water-soluble copolymer. This copolymer is sometimes referred to below as "the polymer of Production Example 4."

### Production Example 5

40 g of acrylic acid, 0.4 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 1.6 g of a block copolymer of 12-hydroxystearic acid and polyoxyethylene (Hypermer B246, produced by Croda) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 39 g of a white fine powder of an alkyl-modified carboxyl-group-containing water-soluble copolymer. This copolymer is sometimes referred to below as "the polymer of Production Example 5."

### Production Example 6

A stirrer, a reflux condenser, and a dropping funnel were attached to a 1000-mL separable flask. In the flask, 48 g of sodium hydroxide was dissolved in 144 g of water. Subsequently, 136.8 g of sucrose was added thereto, and the mixture was stirred at 70 to 85°C for 120 minutes, thereby obtaining an aqueous solution of alkaline sucrose. 145.2 g of allyl bromide was added dropwise to the obtained aqueous solution of alkaline sucrose at 70 to 85°C over the course of 1.5 hours, and then the mixture was reacted at 80°C for 3 hours to allyl-etherify the sucrose. After cooling, 440 g of water was added thereto, and excess oil was removed with a separatory funnel, thereby obtaining an aqueous solution of crude sucrose allyl ether. Hydrochloric acid was added to the obtained crude sucrose allyl ether to adjust the pH to 6 to 8, and water was removed with a rotary evaporator until the mass of the aqueous solution reached 480 g. Subsequently, 200 g of ethanol was added thereto, and salts, such as sodium bromide, (by-products) were precipitated, followed by removing the precipitates from the aqueous solution through filtration. Further, excess water was removed from the aqueous solution with an evaporator, thereby obtaining 166 g of sucrose allyl ether with a degree of etherification of 2.4.

A stirrer, a reflux condenser, and a dropping funnel were attached to a 500-mL separable flask. 72 g of acrylic acid and water were added to the flask to prepare 90 g of an 80 mass% aqueous acrylic acid solution. While this aqueous acrylic acid solution was cooled, 54 g of a 30 mass% aqueous sodium hydroxide solution was added dropwise thereto to prepare an aqueous acrylic-acid-neutralized solution with a degree of neutralization of 40%. Subsequently, 0.32 g of the sucrose allyl ether obtained above and 0.04 g of 2,2'-azobis(2-methylpropionamidine)dihydrochloride (Wako Pure Chemical Industries, Ltd., V-50) were added thereto, thereby preparing an aqueous solution of an ethylenically unsaturated carboxylic acid monomer.

Separately, 330 g of n-heptane was added to a 2000-mL separable flask equipped with a stirrer, a reflux condenser, a dropping funnel, and a nitrogen gas inlet tube, and 2.7 g of sorbitan monostearate (NOF Corporation, NONION SP-60R) was further added thereto, followed by dispersing and dissolving it in n-heptane. Subsequently, the aqueous solution of an ethylenically unsaturated carboxylic acid monomer prepared in advance was added thereto. To remove the oxygen present in the atmosphere in the reaction vessel, in the starting materials, and in the solvent, nitrogen gas was blown into the solution. While the inside of the system was replaced with nitrogen, a reaction was performed for 1 hour with the bath temperature maintained at 60°C, with stirring at a rotational frequency of 1000 rotations/minute. After completion of the reaction, water and n-heptane were distilled off, thereby obtaining a polymer of acrylic acid and a sodium salt thereof. This polymer is sometimes referred to below as "the polymer of Production Example 6."

### Synthesis of Polyalkylene Oxide-Modified Product

### Production Example 7

100 parts by mass of fully dehydrated polyethylene oxide with a number average molecular weight of 20,000, 10 parts by mass of polyoxyethylene polyoxypropylene stearyl ether (EO/PO = 50/50, Mw = 1000), and 0.2 parts by mass of dioctyltin dilaurate were placed in this ratio in a storage tank A equipped with a stirrer kept warm at 80°C. The mixture was stirred in a nitrogen gas atmosphere to obtain a uniform mixture. Separately, dicyclohexylmethane-4,4'-diisocyanate was placed in a storage tank B kept warm at 30°C, and stored in a nitrogen gas atmosphere. Using a metering pump, the mixture in storage tank A and dicyclohexylmethane-4,4'-diisocyanate in storage tank B were continuously fed at a rate of 500 g/min and a rate of 11.9 g/min, respectively, to a twin-screw extruder set to 110 to 140°C (R value = 1.00). The mixture was reacted by mixing in the extruder, and the strand was discharged from the extruder outlet and was formed into pellets by using a pelletizer, thus obtaining a polyalkylene oxide-modified product (nonionic urethane polymer). This polymer is sometimes referred to below as "the polymer of Production Example 7."

### Preparation and Analysis of Viscous Emulsion Composition

Viscous emulsion compositions of the Examples and Comparative Examples were prepared according to the formulations shown in Table 1 by using each copolymer obtained in the Production Examples above. Specifically, an oil component and an emulsifier were measured into a 100-ml plastic beaker and mixed with a Teflon (registered trademark) stirring rod until uniform. The polymer was then added thereto and stirred until uniform, and finally ion-exchanged water was added thereto and stirred until uniform, thereby obtaining a viscous emulsion composition.

The numerical values of each component listed in Table 1 are percentages based on the total mass of all of the components taken as 100%, and the actual preparation was carried out on a scale of 50 g in total.

The viscosity (mPa·s) of each of the obtained viscous emulsion compositions was measured immediately after preparation. Table 1 also show the results. A viscosity immediately after preparation of less than 100000 mPa·s was evaluated as A, a viscosity immediately after preparation of 100000 mPa·s or more and less than 200000 mPa·s was evaluated as B, and a viscosity immediately after preparation of 200000 mPa·s or more was evaluated as C. The viscosity was also evaluated as C when a viscous emulsion composition could not be prepared. Further, the obtained viscous emulsion compositions were allowed to stand for 10 or 13 days after preparation to measure the viscosity in the same manner. Table 1 also show the results. The results confirmed that the obtained viscous emulsion compositions did not lose handleability due to an increase in the viscosity after storage. (Instead, the viscosity tended to decrease, and the handleability was maintained or improved.) The diagonal line in the evaluation columns of the table indicates that no evaluation was made.

After immersing an evaluation sample (each viscous emulsion composition) for 60 minutes or more in a constant-temperature water tank adjusted to 25°C, the viscosity was measured using a Brookfield viscometer (model number: DV1MRVTJ0) at 25°C after 1-minute rotation at a rotation speed of 20 revolutions per minute. For the measurement, a No. 3 rotor was used for viscosity of less than 2000 mPa·s, a No. 4 rotor was used for viscosity of 2000 mPa·s or more and less than 5000 mPa·s, a No. 5 rotor was used for viscosity of 5000 mPa·s or more and less than 15000 mPa·s, and a No. 6 rotor was used for viscosity of 15000 mPa·s or more and less than 40000 mPa·s.

EASYNOV (product name), which was used as an emulsifier, is a composition comprising 50 to 60 mass% of 2-octyldodecanol, 15 to 25 mass% of 2-octyldodecyl xyloside, and 15 to 25 mass% of PEG-30 dipolyhydroxystearate. Information on the other emulsifiers is summarized in Table 2.

**Table 1**

| | Polymer | | Water | Emulsifier | | | | | | | | Oil component | | | | Evaluation | | |
|---|---|---|---|---|---|---|---|---|---|---|---|---|---|---|---|---|---|---|
| | Type | Amount | Ion-exchanged water | Easynov | Rheodol TW-S120V | Rheodol Super TW-0120 | Amphitol 24B | EMAL 20C | SINOLIN SPE-1300 | EMALEX-GWIS-320 | SINOUN SP | Isopropyl myristate | Glyceryl tri(caprylate/caprate) | Jojoba oil | Squalane | Viscosity immediately after preparation | Viscosity 10 days later | Viscosity 13 days later |
| Ex.1 | Polymer of Production Example 1 | 30.0 | 20.0 | 10.0 | | | | | | | | 40.0 | | | | 54800 (A) | | 45000 |
| Ex.2 | Production Example 1 | 25.0 | 25.0 | 10.0 | | | | | | | | 40.0 | | | | 48400 (A) | | 22200 |
| Ex.3 | Polymer of Production Example 1 | 20.0 | 30.0 | 10.0 | | | | | | | | 40.0 | | | | 32800 (A) | | 15000 |
| Ex.4 | Polymer of Production Example 1 | 10.0 | 40.0 | 10.0 | | | | | | | | 40.0 | | | | 4680(A) | | 15000 |
| Ex. 5 | Polymer of Production Example 1 | 25D | 25.0 | 10.0 | | | | | | | | | 40.0 | | | 48800 (A) | | 29800 |
| Ex.6 | Polymer of Production Example 1 | 25.0 | 25.0 | 10.0 | | | | | | | | | | 40.0 | | 53800 (A) | | 37000 |
| Ex.7 | Polymer of Production Example 1 | 25.0 | 25.0 | 10.0 | | | | | | | | | | | 40.0 | 36000 (A) | | 20600 |
| Ex.8 | Polymer of Production Example 1 | 250 | 25.0 | 15.0 | | | | | | | | 35.0 | | | | 77400 (A) | 16,400 | |
| Ex.9 | Polymer of Production Example 1 | 25.0 | 25.0 | 20.0 | | | | | | | | 300 | | | | 167000 (B) | 111800 | |
| Ex.10 | Polymer of Production Example 2 | 25.0 | 25.0 | 10.0 | | | | | | | | 40.0 | | | | 31200 (A) | 18600 | |
| Ex.11 | Polymer of Production Example 3 | 250 | 25.0 | 10.0 | | | | | | | | 40.0 | | | | 56400 (A) | 35800 | |
| Ex.12 | Polymer of Production Example 4 | 25.0 | 25.0 | 10.0 | | | | | | | | 40.0 | | | | 92000 (A) | 69600 | |
| Ex.13 | Polymer of Production Example 5 | 25.0 | 25.0 | 10.0 | | | | | | | | 40.0 | | | | 72800 (A) | 62200 | |
| Ex 14 | Polymer of Production Example 2 | 15/15 | 20.0 | 10.0 | | | | | | | | 40.0 | | | | 11500(A) | | |
| Ex. 15 | Polymer of Production of Production Example 1 | 10/20 | 20.0 | 10.0 | | | | | | | | 40.0 | | | | 37600 (A) | | 30500 |
| Ex. 16 | Polymer of Production Example 6/ Polymer of Production Example 1 | 5/25 | 20.0 | 10.0 | | | | | | | | 40.0 | | | | 56600 (A) | | 39800 |
| Ex. 17 | Polymer of Production Example 6/ Polymer of Production Example 1 | 15/15 | 20.0 | 10.0 | | | | | | | | 40.0 | | | | 36000 (A) | | |
| Comp. Ex. 1 | Polymer of Production Example 1 | 37.5 | 0.0 | 125 | | | | | | | | 50.0 | | | | Dilatancy* (could not be evaluated: C) | | |
| Comp. Ex. 2 | Polymer of Production Example 1 | 40.0 | 10.0 | 10.0 | | | | | | | | 40.0 | | | | Non-uniform (could not be evaluated: C) | | |
| Comp. Ex.3 | Polymer of Production Example 1 | 25.0 | 25.0 | | 10.0 | | | | | | | 40.0 | | | | Aggregated (could not be evaluated: C) | | |
| Comp. Ex.4 | Polymer of Production Example 1 | 25.0 | 25.0 | | | 10.0 | | | | | | 40.0 | | | | Aggregated (could not be evaluated: C) | | |
| Comp. Ex. 5 | Polymer of Production Example 1 | 25.0 | 25.0 | | | | 10.0 | | | | | 40.0 | | | | Aggregated (could not be evaluated: C) | | |
| Comp. Ex.6 | Polymer of Production Example 1 | 25.0 | 25.0 | | | | | 10.0 | | | | 40.0 | | | | Aggregated (could not be evaluated: C) | | |
| Comp. Ex. 7 | Polymer of Production Example 1 | 25.0 | 25.0 | | | | | | 10.0 | | | 40.0 | | | | Aggregated (could not be evaluated: C) | | |
| Comp. Ex.8 | Polymer of Production Example 1 | 25.0 | 25.0 | | | | | | | 10.0 | | 40.0 | | | | Aggregated (could not be evaluated: C) | | |
| Comp. Ex.9 | Polymer of Production Example 1 | 25.0 | 25.0 | | | | | | | | 10.0 | 40.0 | | | | Aggregated (could not be evaluated: C) | | |
| Comp. Ex. 10 | Polymer of Production Example 1 | 25.0 | 25.0 | 5.0 | | | | | | | | 45.0 | | | | Separated (could not be evaluated: C) | | |
| Comp. Ex. 11 | Polymer of Production Example 1 | 25.0 | 25.0 | 2.0 | | | | | | | | 48.0 | | | | Separated (could not be evaluated: C) | | |
| Comp. Ex. 12 | Polymer of Production Example 7 | 25.0 | 25.0 | 10.0 | | | | | | | | 40.0 | | | | Separated (could not be evaluated: C) | | |

| | | | | | | | | | | | | | | | | | | |
|---|---|---|---|---|---|---|---|---|---|---|---|---|---|---|---|---|---|---|
| Dilatancy*: The composition flowed when the container was tilted, but was hardened like a solid when force was applied; thus, the viscosity was unmeasurable. | | | | | | | | | | | | | | | | | | |

**Table 2**

| Emulsifier (labeling name^{*1}) | Reagent name (sales company) |
|---|---|
| Polysorbate 60^{*2} | Rheodol TW-S120V (Kao Corporation) |
| Polysorbate 80^{*3} | Rheodol Super TW-O120 (Kao Corporation) |
| Lauryl betaine/water | Amphitol 24B (Kao Corporation) |
| Sodium lauryl sulfate/water | SINOLIN SP (New Japan Chemical Co., Ltd.) |
| Sodium lauryl sulfate/water | EMAL 20C (Kao Corporation) |
| Sodium lauryl sulfate/water | SINOLIN SPE-1300 (New Japan Chemical Co., Ltd.) |
| PEG-20 glyceryl triisostearate/olive oil | EMALEX-GWIS-320 (Nihon Emulsion Co., Ltd.) |

| | |
|---|---|
| Labeling name^{*1}: Name for the labeling of all ingredients in cosmetics. Polysorbate 60^{*2}: English names: Polyoxyethylene (20) sorbitan monostearate, Polysorbate 60 Other names: Polyoxyethylene sorbitan monostearate, Tween 60 (CAS Number: 9005-67-8) Polysorbate 80^{*3:} English names: Polyoxyethylene (20) sorbitan monooleate, Polysorbate 80 Other names: Polyoxyethylene sorbitan oleate, Tween 80 (CAS Number: 9005-65-6) | |

## Claims

1. A viscous emulsion composition comprising:
(A) at least one copolymer selected from the group consisting of the following (A-1), (A-2), and (A-3), or a salt thereof:
(A-1) a copolymer obtained by polymerizing the following (i) and (ii),
(A-2) a copolymer obtained by polymerizing the following (i) and (iii), and
(A-3) a copolymer obtained by polymerizing the following (i), (ii), and (iii):
(i) 100 parts by mass of a (meth)acrylic acid,
(ii) 0.001 to 1 part by mass of a compound with two or more ethylenically unsaturated groups, and
(iii) 0.5 to 5 parts by mass of a (meth)acrylic acid alkyl ester having an alkyl group with 10 to 30 carbon atoms;
(B) at least one emulsifier selected from the group consisting of the following (B-1), (B-2), and (B-3):
(B-1) an alcohol having a structure in which at least one hydrogen atom of a linear alkane with 18 to 24 carbon atoms is substituted with a hydroxymethyl group or a hydroxyethyl group,
(B-2) an ether of the alcohol (B-1) with a monosaccharide or a polysaccharide of 2 to 6 monosaccharides, and
(B-3) a polyethylene glycol (mono- or di-) ester of a polyhydroxy fatty acid;
(C) water; and
(D) an oil component,
wherein
(A) is present in an amount of 10 to 35 mass%, and the mass ratio ((D)/(B)) of (D) to (B) is 1 to 8.

2. The composition according to claim 1, wherein the mass ratio ((D)/(B)) of (D) to (B) is 2 to 5.

3. The composition according to claim 1 or 2, comprising (D) in an amount of 20 to 50 mass%.

4. The composition according to any one of claims 1 to 3, wherein the composition has a viscosity at 25°C of 1000 to 100000 mPa·s.

5. The composition according to any one of claims 1 to 4, wherein the composition satisfies at least one of the following requirements (α) to (γ):
(α) (B-1) is octyldodecanol,
(β) the saccharide in (B-2) is xylose, and
(γ) (B-3) is a polyethylene glycol mono- or diester of polyhydroxystearic acid.

6. The composition according to any one of claims 1 to 4, wherein
(B-1) is octyldodecanol,
(B-2) is octyldodecyl xyloside, and
(B-3) is a polyethylene glycol diester of polyhydroxystearic acid.

7. The composition according to any one of claims 1 to 6, wherein (A) is selected from the group consisting of the copolymer (A-1) or a salt thereof, and the copolymer (A-3).

8. The composition according to any one of claims 1 to 7, wherein (B) comprises (B-1), (B-2), and (B-3).

9. The composition according to any one of claims 1 to 7, wherein (B) comprises 50 to 60 mass% of (B-1), 10 to 30 mass% of (B-2), and 10 to 30 mass% of (B-3).

10. A viscous emulsion composition comprising:
(A) at least one copolymer selected from the group consisting of the following (A-1) and (A-3), or a salt thereof:
(A-1) a copolymer obtained by polymerizing the following (i) and (ii), and
(A-3) a copolymer obtained by polymerizing the following (i), (ii), and (iii):
(i) 100 parts by mass of a (meth)acrylic acid,
(ii) 0.001 to 1 part by mass of a compound with two or more ethylenically unsaturated groups, and
(iii) 0.5 to 5 parts by mass of a (meth)acrylic acid alkyl ester having an alkyl group with 10 to 30 carbon atoms;
(B) an emulsifier comprising 50 to 60 mass% of the following (B-1), 10 to 30 mass% of the following (B-2), and 10 to 30 mass% of the following (B-3):
(B-1): octyldodecanol,
(B-2): octyldodecyl xyloside, and
(B-3): a polyethylene glycol diester of polyhydroxystearic acid;
(C) water; and
(D) an oil component,
wherein
(A) is present in an amount of 10 to 35 mass%, while (D) is present in an amount of 20 to 50 mass%, and the mass ratio ((D)/(B)) of (D) to (B) is 1 to 8.
